**Europäisches Patentamt**

**European Patent Office**

**Office européen des brevets**

(19)

(11) Veröffentlichungsnummer: **0 331 625 B1**

(12) # EUROPÄISCHE PATENTSCHRIFT

(45) Veröffentlichungstag der Patentschrift:
24.07.91 Patentblatt 91/30

(51) Int. Cl.$^5$: **A61F 2/34**

(21) Anmeldenummer: **89810033.4**

(22) Anmeldetag: **16.01.89**

(54) **Halbkugelförmige Hüftgelenkspfanne.**

(30) Priorität: **01.03.88 CH 770/88**

(43) Veröffentlichungstag der Anmeldung:
**06.09.89 Patentblatt 89/36**

(45) Bekanntmachung des Hinweises auf die
Patenterteilung:
**24.07.91 Patentblatt 91/30**

(84) Benannte Vertragsstaaten:
**AT DE FR GB IT**

(56) Entgegenhaltungen:
**WO-A-85/02535**
**GB-A- 2 137 098**

(73) Patentinhaber: **GEBRÜDER SULZER
AKTIENGESELLSCHAFT
Zürcherstrasse 9
CH-8401 Winterthur (CH)**

(72) Erfinder: **Griss, Peter, Prof. Dr.-med.
Orthopädische Klinik
am Klinikum der Philips-Univ.
Baldingerstrasse
W-3550 Marburg (DE)**
Erfinder: **Koch, Rudolf
Oberdorfstrasse 229
CH-8267 Berlingen (CH)**

## Beschreibung

Die Erfindung betrifft eine halbkugelförmige Hüftgelenkspfanne mit einem unter einem Winkel von 45° aus ihrer Aussenfläche hervorstehenden Verankerungszapfen.

Eine Hüftgelenkspfanne der vorstehend genannten Art ist bekannt aus der EP-A-211 169. Für derartige Pfannen ist in letzter Zeit eine Implantationstechnik entwickelt worden, bei der der Operateur nach der operativen Vorbereitung des Beckenknochens den Zapfen der Pfanne zunächst in die dafür künstlich geschaffene Bohrung verdreht (z.B. ca. 180°) einsetzt und in die richtige Lage hineinschwenkt. Für diese Schwenkbewegung dient der Zapfen als Drehachse, durch die die Halbkugel der Pfanne in ihre korrekte Stellung "geführt" wird.

Aufgabe der Erfindung ist es, durch konstruktive Massnahmen das geschilderte Vorgehen bei der Implantation der Pfanne für den Operateur zu erleichtern und zu vereinfachen. Mit der Erfindung wird diese Aufgabe dadurch gelöst, dass der Verankerungszapfen von einer vom Pfannenkörper getrennten, an ihrem freien Ende haubenartig verschlossenen Hülse umschlossen ist, in der er drehbar gelagert ist.

Die kappenartige Hülse bildet ein technisches Zapfenlager, in dem die Schwenkbewegung wesentlich leichter "läuft", als in der Knochenbohrung ; so wird vor allem eine genaue Einhaltung eines bestimmten Schwenkwinkels ermöglicht. Bei aus Kunststoff gefertigten Zapfen bildet die Hülse, die im allgemeinen aus Metall besteht, zusätzlich eine Abschirmung, durch die direkte Kontakte zwischen Kunststoff und Knochengewebe vermieden werden.

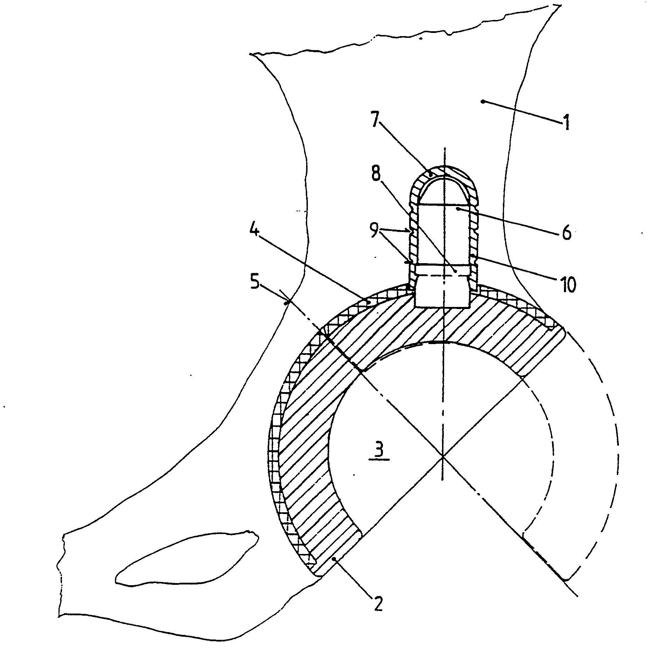

Im folgenden wird die Erfindung anhand eines Ausführungsbeispiels im Zusammenhang mit der Zeichnung näher erläutert.

Die einzige Figur zeigt in der die Zapfenachse enthaltenden Meridianebene schematisch einen Schnitt durch die neue Pfanne, eingesetzt in einen Beckenknochen.

Die in einen Beckenknochen 1 eingesetzte Hüftgelenkspfanne besteht aus einem Kunststoff-Pfannenkörper 2, der die eigentliche Pfannenschale 3 zur Aufnahme eines nicht gezeigten Gelenkkopfes enthält. Auf seiner äusseren Oberfläche ist der halbkugelförmige Pfannenkörper 2 mit einem mehrlagigen Drahtnetz 4 belegt, das durch Einpressen mindestens einer Lage des Netzes 4 in den Kunststoff in bekannter Weise mit dem Pfannenkörper 2 verbunden ist.

Unter einem Winkel von 45° gegen die Polachse 5 des Pfannenkörpers 2 ragt aus der Aussenfläche ein Verankerungszapfen 6 heraus, der vorzugsweise aus Kunststoff besteht und mit dem Pfannenkörper 2 beispielweise durch Reibschweissen verbunden ist. Selbstverständlich ist es auch möglich, den Zapfen 6 aus Metall herzustellen.

Gemäss der vorliegenden Erfindung ist der Zapfen 6 in einer an ihrem freien Ende haubenartig verschlossenen Hülse 7 umgeben, die gegenüber dem Pfannenkörper 2 bzw. dem Drahtnetz 4 frei beweglich ist. Die Verbindung zwischen Zapfen 6 und Hülse 7 besteht in bekannter Weise aus einem Schnappverschluss 8, mit dem der Zapfen 6 beim Einschieben in die Hülse 7 einrastet. Die Mantelfläche des Zapfens 6 und der Hülse 7, die für eine Verankerung im Knochen 1 auf ihrer äusseren Oberfläche strukturiert (Hülle 9) ist, bilden ein zylindrisches Wälzlager 10.

## Patentansprüche

1. Halbkugelförmige Hüftgelenkspfanne mit einem gegenüber ihrer Polachse (5) unter einem Winkel von 45° aus ihrer Aussenfläche hervorstehenden Verankerungszapfen (6), **dadurch gekennzeichnet, dass** der Verankerungszapfen (6) von einer vom Pfannenkörper (2) getrennten, an ihrem freien Ende haubenartig verschlossenen Hülse (7) umschlossen ist, in der er drehbar gelagert ist.

2. Hüftgelenspfanne nach Anspruch 1, **dadurch gekennzeichnet, dass** Zapfen (6) und Hülse (7) ein zylindrisches Wälzlager (10) bilden.

3. Hüftgelenkspfanne nach Anspruch 1 oder 2, **dadurch gekennzeichnet, dass** Zapfen (6) und Hülse (7) durch einen Schnappverschluss (8) miteinander verbunden sind.

## Claims

1. A hemispherical acetabulum having a fixing pin (b) which projects from the outside surface of the acetabulum at an angle of 45° to the polar axis (5) of the acetabulum, characterised in that the fixing pin (6) has extending around it, and is rotatably mounted in, a sleeve (7) which is separate from the prosthetic cup (2) and which is closed after the fashion of a hood at its free end.

2. An acetabulum according to claim 1, characterised in that the pin (6) and sleeve (7) form a cylindrical journal bearing (10).

3. An acetabulum according to claim 1 or 2, characterised in that the pin (6) and sleeve (7) are interconnected by a snap fastening (8).

## Revendications

1. Cotyle hémisphérique de la hanche comportant un pivot d'ancrage (6) dépassant de sa surface extérieure, sous un angle de 45° par rapport à son axe polaire (5), caractérisé en ce que le pivot d'ancrage (6) est entouré d'un manchon (7), fermé à la manière d'un capuchon à son extrémité libre, séparé du corps de cotyle (2), dans lequel il est monté pivotant.

2. Cotyle de la hanche selon la revendication 1, caractérisé en ce que le pivot (6) et le manchon (7) forment un palier de roulement (10) cylindrique.

3. Cotyle de la hanche selon la revendication 1 ou 2, caractérisé en ce que le pivot (6) et le manchon (7) sont reliés entre eux par une fermeture à encliquetage (8).